# EUROPEAN PATENT APPLICATION

(11) **EP 2 322 191 A2**
(43) Date of publication of application: **18.05.2011**
(21) Application number: 09803139.6
(22) Date of filing: 29.07.2009
(51) Int. Cl.: A61K 35/12, A61K 35/00, C12P 21/08, A61K 38/00, A61K 39/00

(54) **SENESCENCE CONTROL COMPOSITION CONTAINING EXTRACELLULAR MATRIX COMPONENTS, AND SENESCENCE CONTROL METHOD FOR SENESCENT CELLS USING SAME**

(30) Priority: 31.07.2008 KR 20080075061
(71) Applicant: SNU R&DB Foundation, Gwanak-gu Seoul 151-015 (KR)
(72) Inventor: PARK, Sang Chul, Seongnam-si Gyeonggi-do 463-470 (KR); CHO, Kyung A, Seoul 150-935 (KR); HA, Moon Kyung, Seoul 151-015 (KR); CHOI, Hae Ri, Seoul 158-820 (KR)
(74) Representative: Katzameyer, Michael
(86) International application number: PCT/KR2009/004212
(87) International publication number: WO 2010/013934

(57) **Abstract**

The present invention relates to a composition containing an extracellular matrix for controlling aging and a method for controlling aging by using the same. The composition for controlling aging and the method for controlling aging have an effect for recovering the biological function of the aged aging cell, and the amount of the expression of the integrin alpha V that is a cell membrane protein and is reduced if aging is again increased in the aging cell recovered by the young extracellular matrix. However, when the amount of expression of the integrin alpha V is artificially decreased by using siRNA and antibody, the aging cell is not recovered by the extracellular matrix extracted from the young cell.

## Description

### [Technical Field]

The present invention relates to a senescence control composition containing extracellular matrix components, and senescence control method for senescent cells using the same.

### [Background Art]

A human fibroblast moves to the stage of aging cell, in which a cell is not divided anymore, after a cell division (1-5). The aged cells will occur various changes. Examples of the various changes of aged cells include, for example, the increase of molecules that disrupt a cell cycle (6-8), the change of shape (9-10), the increase of betagalactosidase that is a marker for aging cell (11), the accumulation of the oxidative damages, the decrease of reactivity about the factors that stimulate a growth (12), the increase of resistance about a cell death (9), and the like, and when accumulating the above changes, the functions are decreased thereby progressing aging.

A cell affects the surrounding cells as well as itself by environmental factors such as an extracellular matrix that is extracted. The extracellular matrix is composed of a collagen, an elastin, a proteoglycan, and the like. The extracellular matrix affects the cellular organization of tissue and various activities of cell. It is reported that the constituents of extracellular matrix are changed during the process of aging (13) and are changed according to the disease associated with aging, such as Type 2 Diabetes, Werner Syndrome, and the like (14, 15). It suggested that receptive and un-receptive factors that are isolated from the aging cell are involved in the canceration of aged organism (16). The activated extracellular matrix affects the destiny of cell. In addition, the factors that are extracted from the aging fibroblast affect the canceration and growth of an epithelial cell (17). However, not much is known about the effect on the destiny of young cell and aged cell until now.

The relationship between cell and extracellular matrix is modulated by an integrin that is one of cell-coupled receptors. The cell and extracellular matrix modulated by the integrin affect the growth, movement, damage and survival of cell (18). The integrin is composed of 18 alpha chains and 8 beta chains, and 24 integrins that are different to each other are constituted through the various bindings. The various intengrins that are formed by the above ways have a binding specificity per cell (19). It is known that the integrin alpha V among them has an important role on forming cancer by binding to the fibronectin and vitronectin extracellular matrix (20). According to the recent researches, it is known that the integrin alpha V largely influences the damage and growth of a cancer, such as a melanoma, a breast cancer, and the like (21, 22).

### [Disclosure]

### [Technical Problem]

For this reason, the inventors completed the present invention by finding the facts that the functional damage and structural damage are generated by the extracellular matrix that is extracted from the aged human fibroblast, and also the function of aged cell that is already aged is recovered by the extracellular matrix that is extracted from the young cell while the inventors were trying to find the factors that can control aging.

Thus, the technical object of the present invention is to provide a composition containing an extracellular matrix component for controlling aging.

In addition, the object of the present invention is to provide a method for controlling aging by using the composition.

Other objects and advantages of the present invention will be described in more detail with reference to the following description along with the accompanying claims and drawings.

In addition, many cited references and patent documents would be referenced and the quotations would be marked over all the description. The disclosed contents in the cited references and the patents would be inserted as a reference in the description as a whole so that the content of the present invention and the level of the technical field, to which the present invention belongs, would be described in more clearly.

### [Technical Solution]

According to an embodiment of the present invention, the present invention provides a composition containing an extracellular matrix component for controlling aging.

The term, "senescene" used in the description has the same meaning as aging. Thus, "senescense control" means the entire phenomenon for controlling aging. As more specific examples, it means that the biological function of aged cell is recovered so that a similar biological phenomenon as a young cell is appeared. For example, for the aging cell that is treated with the composition according to the present invention, the reactivity about a growth factor, such as EGF (Epithelial growth factor), is recovered, so that the signal transduction is recovered by the growth factor and the cell cycle is normally operated. In addition, it includes the phenomenon of accelerating aging or inducing aging. For example, it includes the phenomenon that aging is induced thereby slowing the number of cell division, or the biological phenomenon similar to the aging cell is appeared.

According to an embodiment of the present invention, the recovery of division ability or aging of cell that is cultured in a medium containing an extracellular matrix of young cell can be analyzed. More specifically, after the young cell and aging cell of the human fibroblast are cultured in a culture dish containing the medium without serum for 2-3 days, the cells only are removed from the culture dishes and the extracellular matrix in culture dish is obtained. Since then, the young cell or aging cell is seeded in culture dishes containing the extracellular matrixes of young cell or aging cell, respectively. The cell is cultured under the conditions for total four groups, such as 'the medium containing the extracellular matrix of young cell + young cell, the medium containing the extracellular matrix of aging cell + young cell, the medium containing the extracellular matrix of young cell + aging cell and the medium containing the extracellular matrix of aging cell + aging cell,' and then the growth of each cell may be observed and analyzed to analyze whether or not the division ability or aging is recovered. In addition, it can be identified that the medium containing the extracellular matrix, i.e., composition can control aging of cell. In addition, it can be identified that the recovered division ability of aging cell is caused by the cancer modification of normal cell or is simply a recovery of the normal division ability.

According to a specific example of the present invention, the aging control by the composition containing the extracellular matrix can be identified by analyzing the beta galactosidase reaction that is well known as a marker of aging cell, the degree of oxygen activation, F-actin staining degree, the quantitative change of p21^{Waf1} and p16^{INK4a} that are defense molecules of the cell cycle that are well known for controlling the cell division of aging cell, a change of the amount of cabeoline expression that is involved in the cell cycle, the binding degree and the amount of expression of integrin alpha V that is a binder with the fibronectin that is an extracellular matrix, and the like.

According to a specific embodiment of the present invention, the functional damage and the structural change of cell in the human fibroblast occurs due to the extracellular matrix that is produced by aging the human fibroblast. In addition, when the aging cell is cultured in the medium containing the extracellular metrix component that is derived from the young cell, the division ability of the above aging cell is recovered as much as the division ability of young cell and even the physiological function of the above aging cell is changed as similar as that of young cell. At this time, the amount of integrin alpha V expression is reduced, but is recovered by the extracellular matrix components of young cell, in which the integrin alpha V is a kind of the cell membrane proteins that bind to the fibronectin, the vitronectin, and the like among the extracellular matrix components. However, when the degree of activity and the amount of integrin alpha V expression are artificially reduced by antibody or siRNA about the integrin alpha V, the division ability or aging in the aging cell is not recovered.

Thus, the present invention is characterized by containing the extracellular matrix component as the composition for controlling aging.

According to the present invention, the object for aging is a mammal.

The extracellular matrix component suitable for the present invention may be obtained by culturing the cell of mammal, preferably from a fibroblast that is derived from a mammal, and more preferably from a fibroblast that is derived from human.

In addition, the extracellular matrix component includes all of components that are derived from the young cell and aging cell, but most preferably the components that are derived from the young cell. According to the results of preferable embodiments, when the aging cell, of which the growth almost ends, is cultured in the culture dish containing the extracellular matrix component obtained from the young cell, the shape and features (for example, the division ability, and the like) of the aging cell are recovered as similar as that of the young cell.

According to the present invention, the composition for controlling aging is preferably used by mixing with the culture medium for culturing cell, and other components in addition to the extracellular matrix components may further be added thereto.

According to the preferable embodiment of the present invention, the recovery of the biological function of aging cell is suppressed by adding antibody or siRNA about the integrin alpha V to the composition for controlling aging. From the above result, it can be strongly shown that the recovery of the biological function of the aging cell is involved in the integrin alpha V.

Thus, the present invention provides the composition for controlling aging, in which the composition comprises one of the material for suppressing the expression and activity of the integrin alpha V by binding to DNA, RAN or protein of the integrin alpha V, such as a double-stranded siRNA that suppresses the expression of integrin alpha V and has a complementary sequence to mRNA of the integin alpha V, the antibody to the integrin alpha V that suppresses the activity of the integrin alpha V by binding the integrin alpha V that is in the cell membrane, a ligand (antagonist), and a inhibitor.

The composition according to the present invention may be made as a dosage form for a cosmetic, a pharmacy, and a healthy food or drink through the general method that is well known in the relevant field.

When the composition according to the present invention is prepared as the composition for a cosmetic, the composition comprises the components that are generally used for the cosmetic composition in addition to the extracellular matrix component as the above effective component, and more specifically comprises common adjurvants, such as an antioxidant, a stabilizer, a solubilizer, a vitamin, a pigment and flavoring, and a carrier. The cosmetic composition according to the present invention can be prepared as any types of dosage forms that are generally formed in the art, and for example can be prepared as a dosage form, such as a solution, a suspension, an emulsion, a paste, a gel, a cream, a lotion, a powder, a soup, a surfactant-containing cleansing, an oil, a powder foundation, an emulsion foundation, a wax foundation, a spray, and the like, but is not limited thereto. More specifically, the cosmetic composition according to the present invention can be prepared as a dosage form, such as an astringent, a nutrient tonic, an nourishing cream, a massage cream, an essence, an eye cream, a cleansing cream, a cleansing form, a cleansing water, a pack, a spray or a powder.

When the composition according to the present invention is prepared as a pharmaceutical composition, the composition may generally comprising a pharmaceutically acceptable carrier in addition to the extracellular matrix component, and more specifically it may comprising a carbohydrate-based compound (examples: lactose, amylose, dextrose, sucrose, sorbitol, mannitol, starch, cellulose, and the like), an acacia rubber, calcium phosphate, alginate, gelatin, calcium silicate, microcrystalline cellulose, polyvinylpyrrolidone, cellulose, water, syrup, salt solution, alcohol, arabia rubber, vegetable oil (examples: corn oil, cotton seed oil, soybean oil, olive oil, coconut oil), polyethylene glycol, methyl cellulose, methyl hydroxy benzoate, propyl hydroxy benzoate, talc, stearate magnesium, mineral oil, and the like, but is not limited thereto. The composition according to the present invention may further include a lubricant, a wetting agent, a sweeting agent, a flavor, an emulsifier, a suspension, preservatives, and the like in addition to the above components.

The composition according to the present invention may be prepared in a type of unit capacity or in putting within a multicapacity container by formulating using a pharmaceutically acceptable carrier and/or an excipient according to the method that can be easily performed by the person who has common knowledge in the field that includes the present invention. At this time, the dosage form may be a type of solution, suspension or emulsion in an oil or aqueous medium, or a type of extract, powder, granular, tablet or capsule. The composition according to the present invention may include a buffer solution that is dissolved with an adequate amount of salt or pH regulator in order to maintain a physiological activity of an effective component as efficiently as possible. In addition, the composition according to the present invention may be administrated by further including a dispersing agent or stabilizer in order to effectively apply the effective component according to the present invention.

The suited pharmaceutically acceptable carrier and pharmaceutical preparation are described in more detail in Remington's Pharmaceutical Sciences (19th ed., 1995).

The pharmaceutical composition according to the present invention may be orally or parenterally administrated. When administrating parenterally, it may be administrated by a type of intravenous infusion, subcutaneous transfusion, muscular injection, intraperitoneal infusion, transdermal administration, and the like.

The suitable dosage of the pharmaceutical composition according to the present invention may vary depending upon the factors, such as a way of formulation, a way of administration, a patient age, a patient body weight, a patient sex, a condition of patient food, an administration time, an administration route, an elimination speed, sensitivity to the reaction, and the like. A doctor who is skilled generally can easily decide and prescribe the effective dosage for the desired prevention or treatment, and the dosage may be decided by dividing into one time or several times per a day.

According to the other embodiment of the present invention, the present invention provides a method for controlling aging, comprising treating with the composition containing an extracellular matrix component.

In addition, the present invention provides a method for controlling aging, comprising treating with the composition containing the inhibitor, the ligand (antagonist), the antibody or siRNA to the integrin alpha V.

According to the preferable embodiment of the present invention, the extracellular matrix component is derived from a young cell, more preferably from the young cell of human, and most preferably from the human fibroblast. In addition, the object for controlling aging is a animal, preferably a mammal, and most preferably the human fibroblast.

The duplicated content in the method according to the present invention and the composition according to the present invention as disclosed above will not be described in order to avoid the complexity of the description. In addition, the technical and scientific terms that are used in the description have the meanings similar to the meanings that are generally understood by the person who has general knowledge in the art unless they are not particularly defined.

### [Advantageous Effects]

The present invention relates to a composition containing an extracellular matrix component for controlling aging. In addition, the present invention relates to a method for controlling aging using the above composition. The composition for controlling aging and the method for controlling aging according to the present invention has an effect on recovering the biological function of the aging cell that is already aged, and the amount of the expression of the integrin alpha V that is a reduced cell membrane protein while processing aging is again increased in the aging cell recovered by the young extracellular matrix.

### [Description of Drawings]

The above and other objects, features and advantages of the present invention will become apparent from the following description of preferred embodiments given in conjunction with the accompanying drawings, in which:

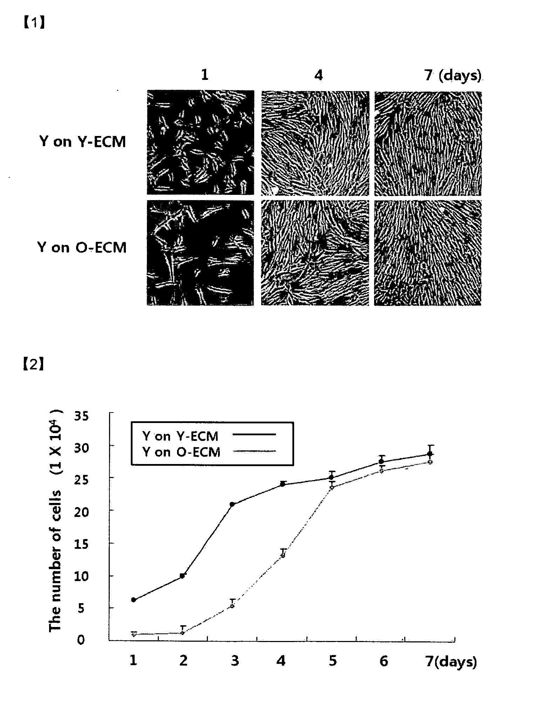

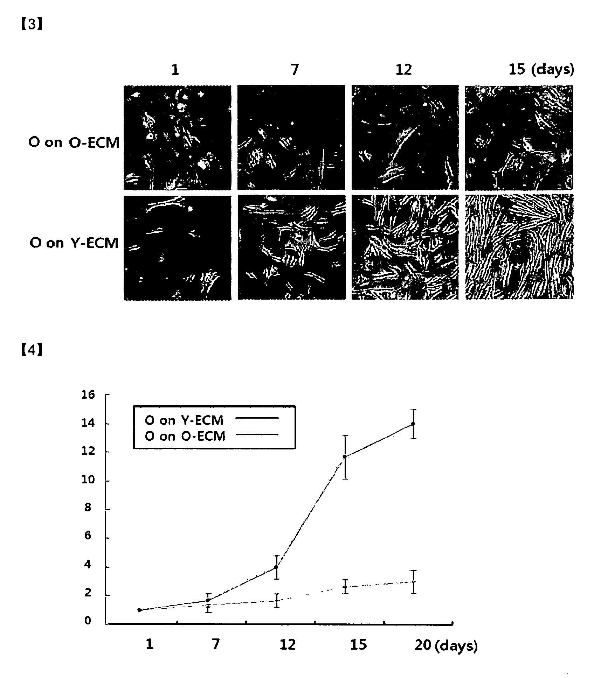

Fig. 1 to Fig. 4 relates to the growth ability of the aging cell and young cell depending upon the extracellular matrix. After the young cells were cultured in the extracellular matrix extracted from the aging cell and the extracellular matrix extracted from the young cell for 7 days, and then the cells were observed. Fig. 1 is the photographs showing the young cells that were added into the aging extracellular matrix and young extracellular matrix observed from a microscope per days that were disclosed in the figure, i.e., 1, 4 and 7 days. Fig. 2 is a graph showing the number of cells as disclosed in Fig. 1. Fig. 3 is the photographs showing the cells observed through a microscope after the aging cells were added into the extracellular matrix obtained from the young cell and the extracellular matrix obtained from the aging cell. Fig. 4 is a graph showing the number of cells as disclosed in Fig. 3. Y = Young Cell; O = Aging Cell; Y-ECM = Extracellular matrix extracted from the young cell; O-ECM = Extracellular matrix extracted from the young cell.

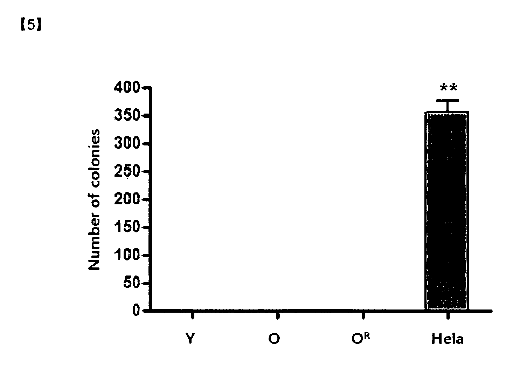

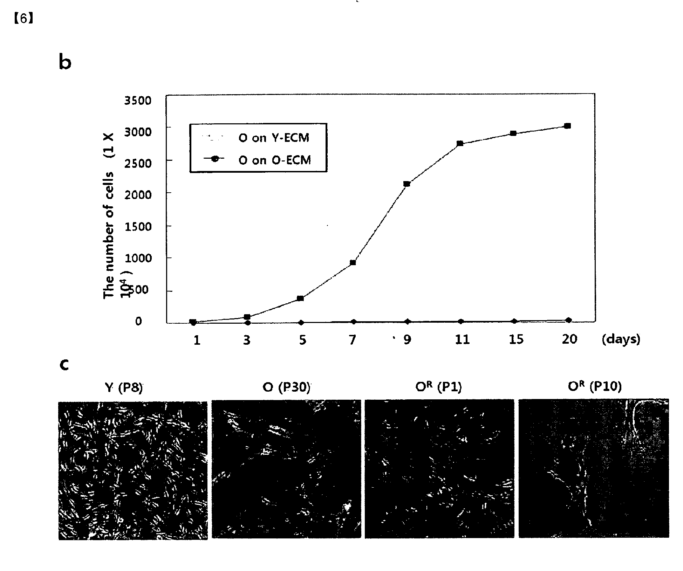

Fig. 5 to Fig. 6 show the effect and growth ability of the aging cell by the young extracellular matrix. Fig. 5 shows the results of the reaction experiment for forming a colony by using, Hela cell (cancer cell), a young cell, an aging cell, and the aging cell that is recovered by the young extracellular matrix. The bar graph shows the number of average colonies. An asterisk indicates P < 0.01 by Student's t test. Fig. 6b shows the growth curve of the aging cell that was added into the aging extracellular matrix and the young extracellular matrix. Fig. 6c shows the results of staining of beta-galactosidase of the young cell, the aging cell, the recovered aging cell, and the aging cell of the recovered aging cell. P = Subculture; OR = Recovered aging cell; ORS = Aging cell of the recovered aging cell.

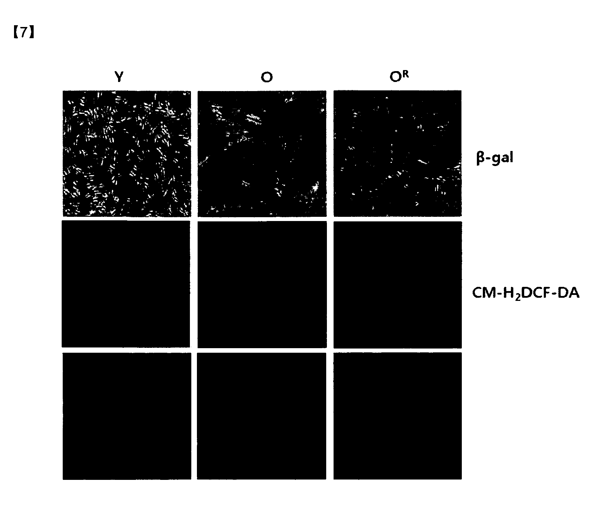

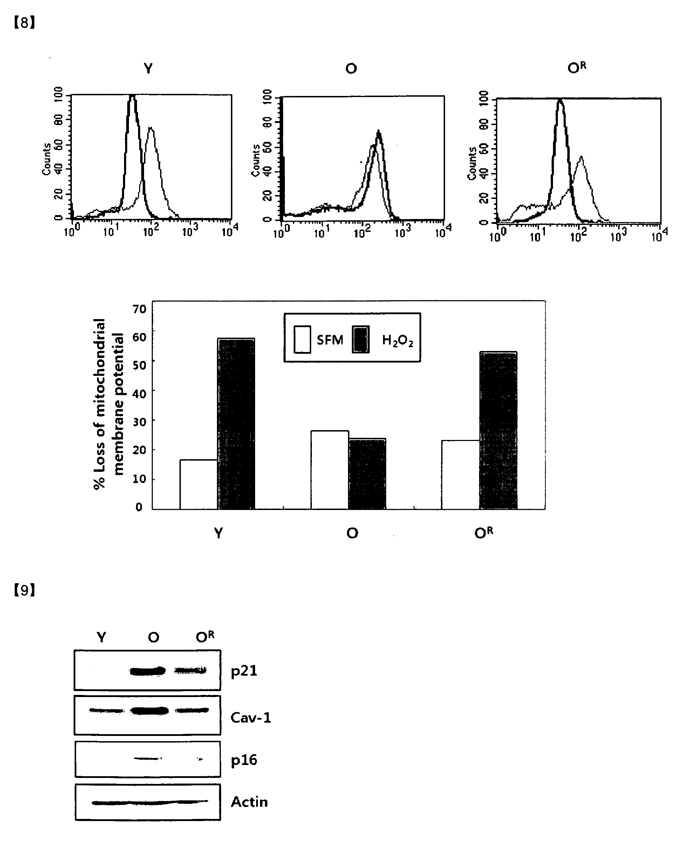

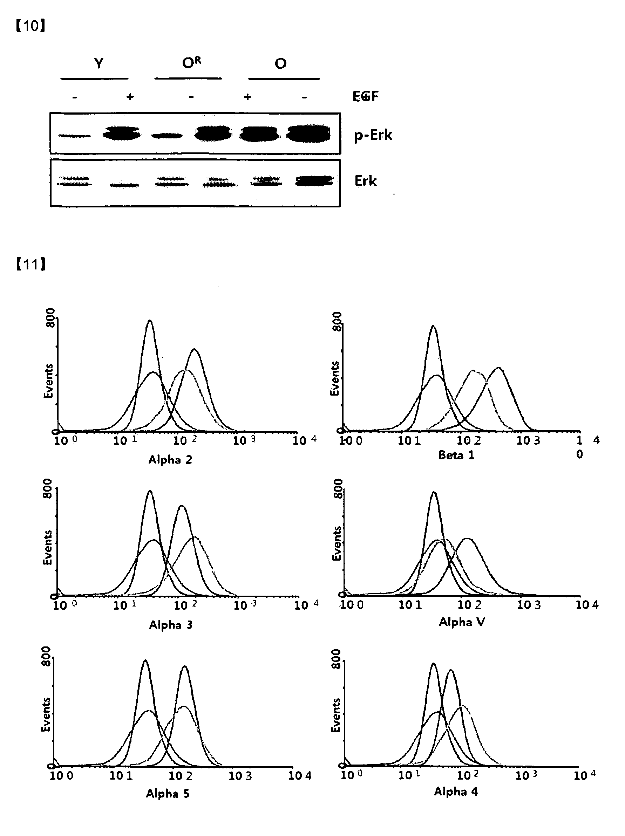

Fig. 7 to Fig. 10 are the features of the recovered aging cell. Fig. 7 shows the results of beta-galactosidase staining (first Panel), CM-H2DCF-DA staining (Second Panel), DAPI and F-actin staining (Third Panel). Fig. 8 shows the membrane potentials in the young cell, the aging cell and the recovered aging cell (Black; Medium without serum and antibiotic; Red; Medium with H2O2). Fig. 9 shows the results of identifying the amount of expression of p21, p16 and caveolin in the young cell, the aging cell and the recovered aging cell through Western Blot. Fig. 10 shows the results of identifying the amounts of protein expressions of Erk that were phosphorylated in the young cell, the aging cell and the recovered aging cell that were treated with 100 ng/ml of EGF for 30 minutes through Western Blot. CM-H2DCF-DA = 5-(and-6)-chloromethyl-2', 7'-dichlorodihydrofluoresceindiasetate, acetylester

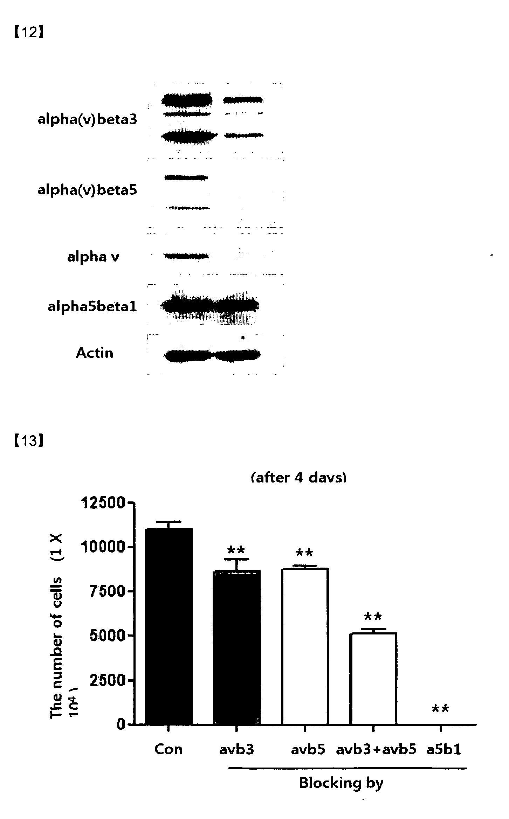

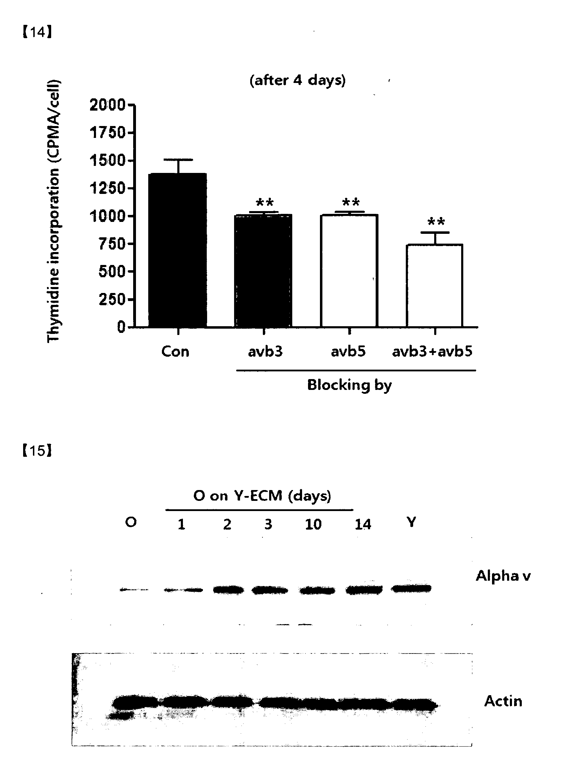

Fig. 11 to Fig. 14 are the degree of expression of integrin of the young cell and the aging cell. Fig. 11 shows the results of comparing the degrees of expressions of integrin in the young cell and the aging cell through the flow cytometery (Black, Young cell; Blue, Young cell bound with integrin antibody; Red, Aging cell; Green, Aging cell bound with integrin antibody). Fig. 12 shows the results of identifying the amounts of expressions of the young cell and the aging cell by using alpha (v) beta3, alpha (v) beta5, alpha (v) and alpha5beta1 antibodies through Western Blot. Fig. 13 and Fig. 14 show the results of the degree of synthesizing DNA and the number of cell after adding the integrin antibody into the young cell and the aging cell. A bar graph uses ANOVA program and an asterisk indicates P < 0.01. Con = Control; avb3 = Integrin alpha (v) beta3 antibody; avb5 = Integrin alpha (v) beta5 antibody; avb3 + avb5 = Adding alpha (v) beta3 antibody and integrin alpha (v) beta5 antibody altogether; a5b1 = Integrin alpha5beta1 antibody.

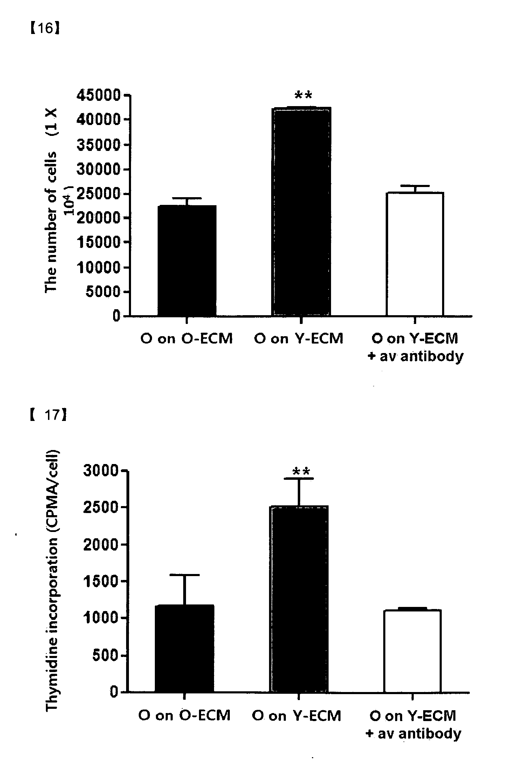

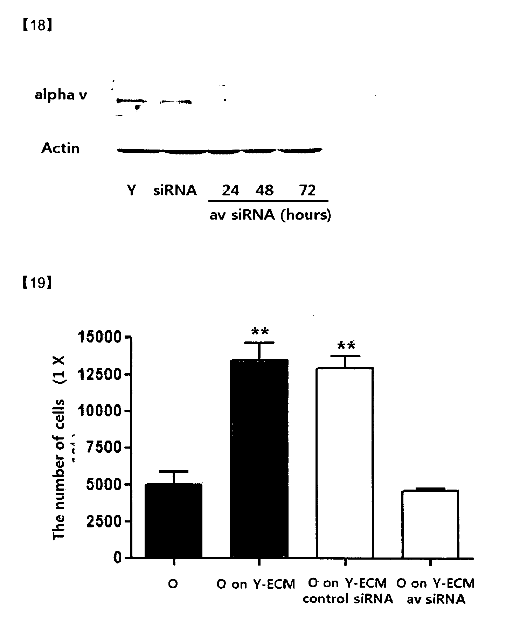

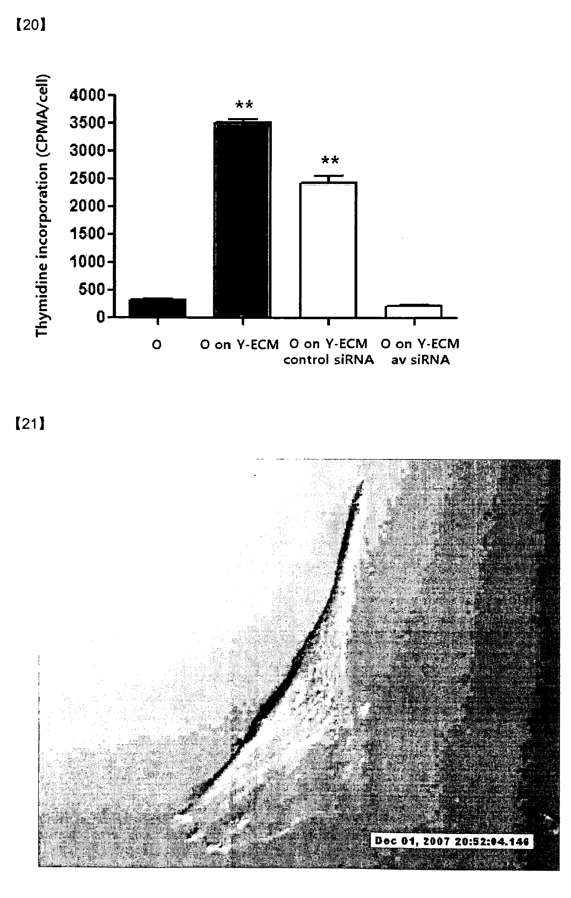

Fig. 15 to Fig. 20 show the expressions of the integrin alpha V in the process of recovering the aging cell that was added into the young extracellular matrix. Fig. 15 shows the result of comparing the amounts of expressions of the integrin alpha V of the gaing cells that were added into the young extracellular matrix through Western Blot per hour. Fig. 16 shows the number of cells in the aging cell under the different conditions (the aging cell in the aging extracellular matrix, the aging cell in the young extracellular matrix, the aging cell in the young extracellular matrix that is added with the integrin alpha V antibody) and Fig. 17 shows the degree of synthesizing DNA in the aging cell under the different conditions (the aging cell in the aging extracellular matrix, the aging cell in the young extracellular matrix, the aging cell in the young extracellular matrix that is added with the integrin alpha V antibody). Fig. 18 shows the young cells that were added with the integrin alpha V siRNA through Western Blot. Since then the aging cell was added into the above conditions, and cultured in the culture dish containing the young extracellular matrix. Fig. 19 shows the number of cells that were recovered after 10 days and Fig. 20 shows the degree of synthesizing DNA. A bar graph shows the average values and error bar indicates a standard deviation. An asterisk indicates P<0.01 by Student t test.

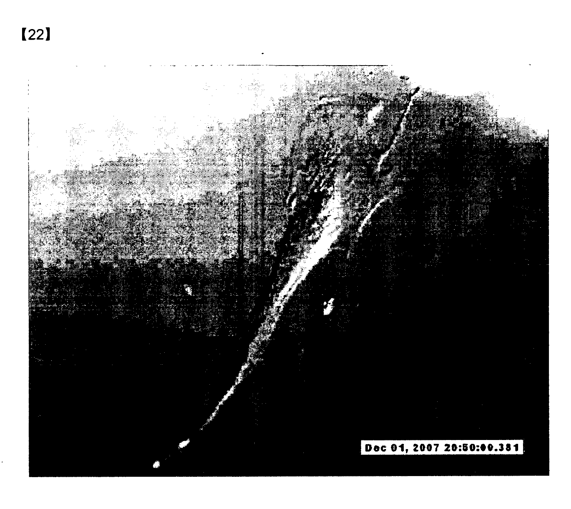

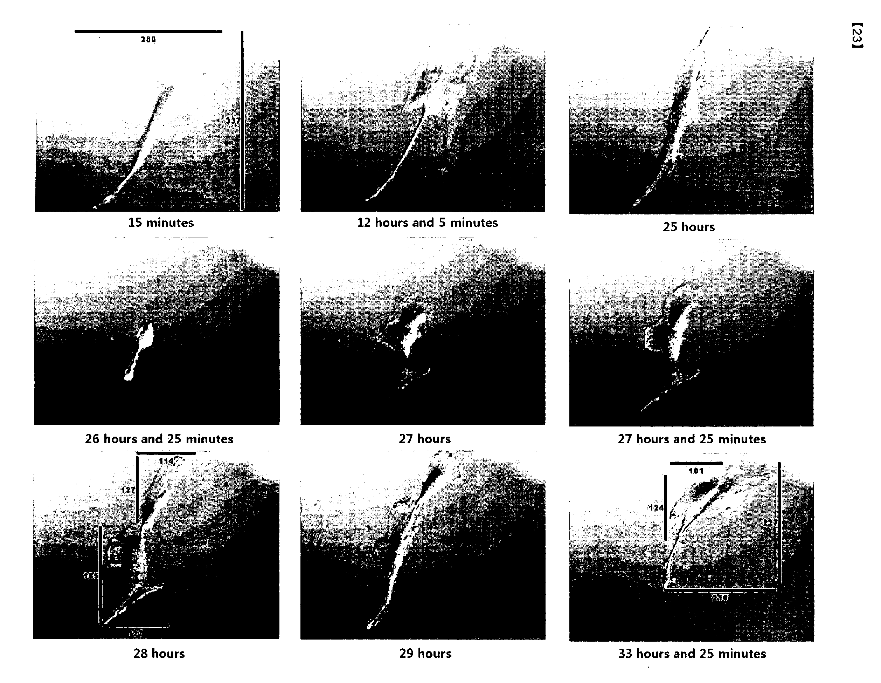

Fig. 21 to Fig. 23 show the cell images by using Time Lapse. The aging cell in the young extracellular matrix as disclosed in Fig. 21 and the aging cell in the aging extracellular matrix as disclosed in Fig. 22 were compared through Time Lapse. Fig. 23 shows the snap pictures of aging cell in the young extracellular matrix per hour.

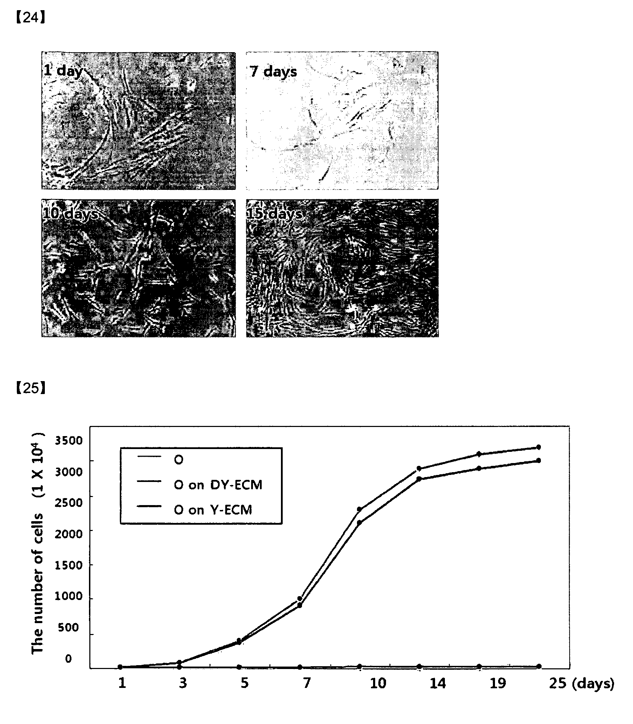

Fig. 24 and 25 show the recovery of the aging cell by the extracellular matrix obtained from the young cell of other object, in which the aging cell was added into the young extracellular matrix obtained from other object by using the conventional method. Fig. 24 shows the photographs of cell change through a microscopy. Fig. 25 shows the photographs of lifespan of the aging cells obtained by the young extracellular matrix obtained from the same object as the aging cell and the young extracellular matrix obtained from the object different from the aging cell.

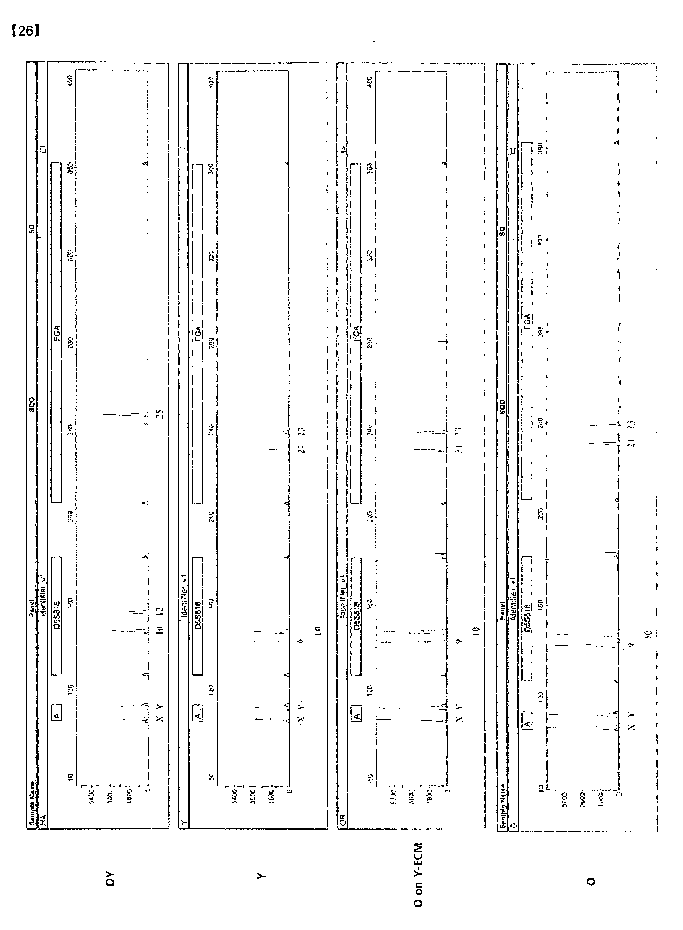

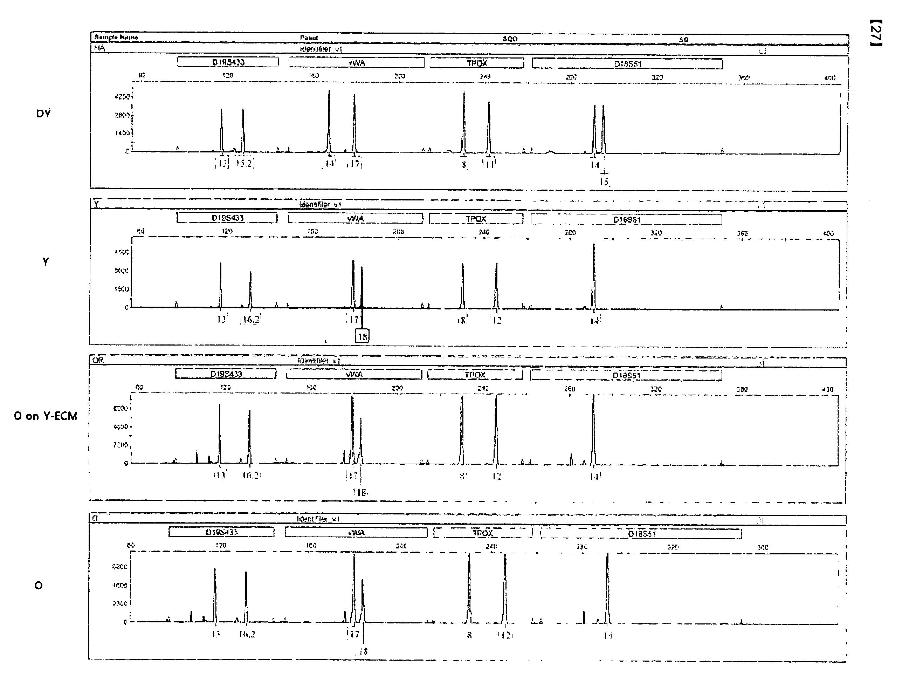

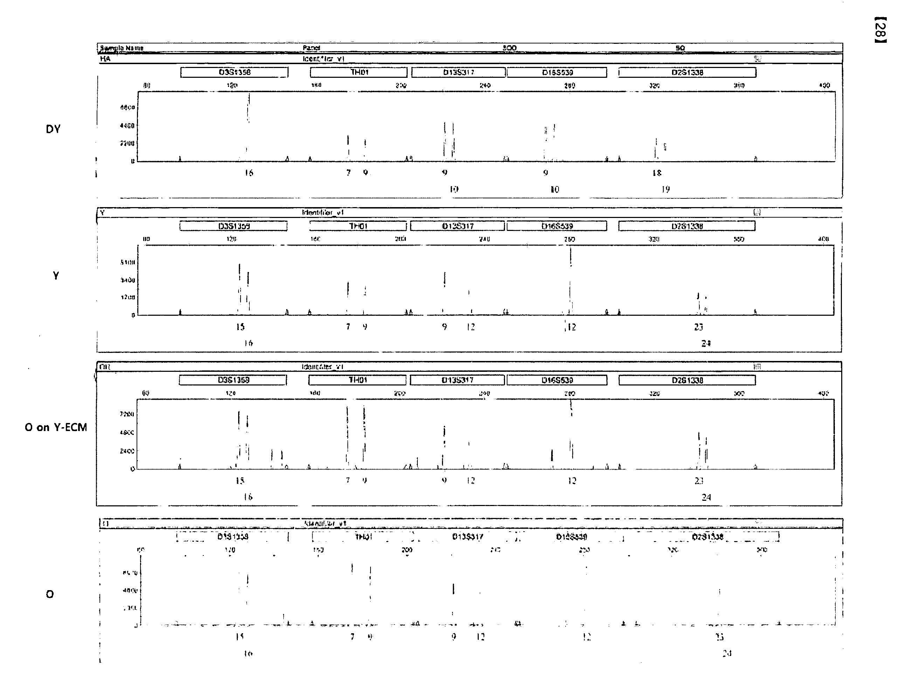

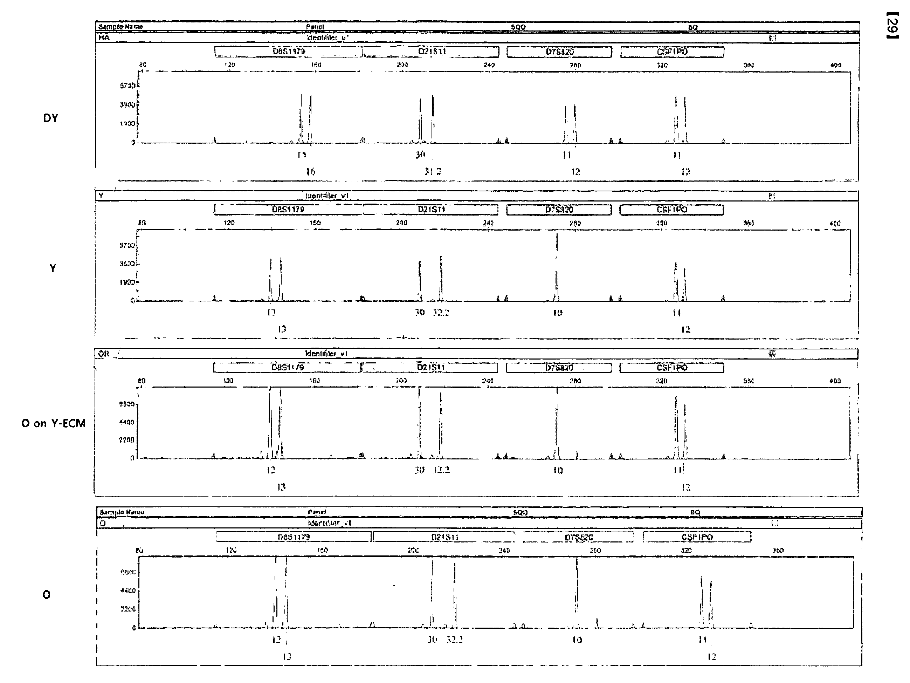

Fig. 26 to Fig. 29 show the recovery of aging cell by the extracellular matrix obtained from the young cell of different object, and the results of comparing with 15 STR loci through DNA fingerprinting method with the young cell, the aging cell, the recovered aging cell, and the young cell obtained from the different object (D8S1179, D21S11, D7S820, CSF1PO, D3S1358, TH01, D13S317, D16S539, D2S1338, D19S433, vWA, TPOX, D18S51, D5S818, FGA). O = Aging cell obtained from the subculture with the epithelial cell obtained from the foreskin of 6-year old boy; DY-ECM = Extracellular matrix obtained by the foreskin cell of newborn baby.

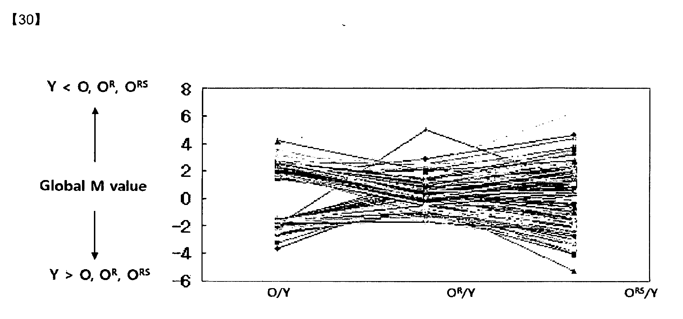

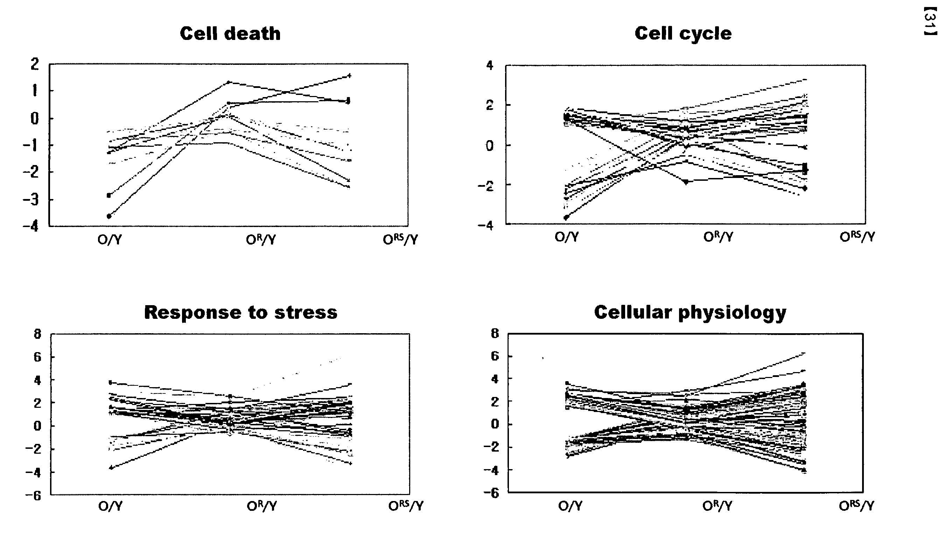

Fig. 30 and 31 show the results of gene comparison, in which the results were obtained by comparing the genes of the young cell, the aging cell, the recovered aging cell and again recovered aging cell by using CodeLink Bioarray (uniest Human 20K. The results mark the measured Global M value, (-) indicates the reduced gene when the young cell was used and (+) indicates the increased gene when the young cell was used.

### [Best Mode]

Hereinafter, the embodiments of the present invention will be described in detail with reference to accompanying drawings. The embodiments are given by way of illustration only for describing the present invention in more detail, and it will be understood by the person who has general knowledge in the art such that the point of the present invention will not be limited to the above embodiments according to the point of the present invention.

### <Example>

### Example 1. Preparation of Materials: Antibodies, Reagents and Chemical Agents.

The antibodies used for the present invention were purchased from the following companies. Caveolin-1 monoclonal antibody was purchased from BD transductions company (Palo Alto, CA), and Integrin Alpha V (MAB2021Z), Integrin Alpha (V) beta3 (MAB1976), Integrin Alpha (V) beta5 (MAB1961) and Integrin Alpha5beta1 (MAB1969) antibodies were purchased from Chemicon international company. Phospho-Erk polyclonal antibody (sc-7383), Erk-1/2 antibody (sc-94), p53 antibody (sc-126), and p21 antibody (sc-6242) were purchased from Santa Cruz Biotechnology, Inc (SantaCruz, CA). Horseradish peroxidase-linked secondary antibody of rat and rabbit were purchased from Zymed Laboratories, Inc. (SanFrancisco, CA).

Chemiluminescent detection reagent was purchased from Pierce Company (Rockfor, IL). β -actin monoclonal antibody and hydrogen peroxide(216763) were purchased from Sigma Chemical Co (St. Louis, MO, USA). DiOC₆ (3,3-diethyloxacarbocyanine) and CM-H₂DCFDA (5-(and-6)-chloromethyl-2,7-dichlorodihydrofluoresceindiacetate, acetylester) were purchased from Molecular Probes Company. *[methyl-³H]* thymidine was purchased from Amersham Biosciences Company.

### Example 2. Preparation of Cell Culture and Extracellular Matrix

The new young fibroblast was obtained from the newborn baby and the young human fibroblast was extracted from the foreskin of 6-year boy. The above cells were sub-cultured above 60 times so that the aging was induced and the subculture method was performed by using a general culture method. Briefly, the cell was cultured in the culture dish having 100 mm of diameter containing Dulbecco's modified Eagle's medium containing 10 % bovine fetal serum, 100 units/ml of penicillin, and 100 ug/ml of streptomycin at the incubator of 37 °C and 5 % CO₂ conditions. The subculture was performed as follows: when the occupation percent of cell in the bottom of culture dish becomes above 70 to 80 %, the cells were isolated from the culture dish by using 0.5 % trypsin-EDTA and then transferred into the new culture dish containing the culture medium. The aging of cell was identified by identifying whether beta galactosidase staining is reacted or not, and the degree of synthesizing DNA. The extracellular matrix was obtained by culturing the cell in the medium without serum for 2-3 days, removing only cells by using EDTA, and then clearly washing the cell. The culture dish containing the obtained extracellular matrix was stored at 4 °C for at least one day and then used.

### Example 3. Beta Galactosidase Staining and CM-H2CFDA Staining relevant to aging

The human fibroblast was washed with PBS and then fixed with 4 % of paraformaldehyde for 5 minutes. The cells was washed with PBS and then added into the betagalactosidase staining solution (1 mg/ml 5-bromo-4-chloro-3-indolyl-β -D-galactopyranoside (X-gal), 40 mM citric acid/sodium phosphate-pH 6.0, 5 mM potassium ferrocyanide, 5 mM potassium ferricyanide, 150 mM NaCl, 2 mM MgCl₂) to stain at the incubator of 37 °C. Since then, it was identified whether the beta galactosidase staining was occurred or not by using a microscope.

After the human fibroblast was grown to about 70 %, the fibroblast was added into 1 uM CM-H2DCF-DA (5-(and-6)-chloromethyl-2,7'-dichlorodihydrofluoresceindiacetate, acetylester) and then reacted at 37 °C for 15 minutes. Since then the cell was observed through the fluorescence microscope.

### Example 4. Cell Time Lapse Microscope

In order to collect the extracellular matrix, the young cell was cultured in the medium without serum at Lab-Tek II Chamber Slide w/Cover CC2 Glass Slide Sterile (Nalge Nunc international Company). After 3 days, the extracellular matrix was obtained by removing the cells using EDTA. The same number of aging cells were added into the culture dish containing the young extracellular matrix and then observed with a microscope (Olympus 1X81) for 10 days. The photographs were analyzed with Meta Image Series Software (Molecular Devices, ied Biosystems).

### Example 5. Immunefluorescence Analysis

The cell in the cover glass with 12 mm(φ) diameter was washed with PBS and then fixed with 4 % paraformaldehyde for 10 minutes. And then, the cell was treated in 0.5 % Triton X-100 solution for 10 minutes for the transmission of cell. In order to avoid binding with the undesired protein, the cell was treated in 2 % bovine serum albumin for 30 minutes. After the cell was washed with PBS and then reacted with Actin monoclonal first antibody, the unbound proteins were removed. The cell was reacted with Texas Red (Molecular Probes and Santa Cruz Biotechnology, Inc.) for 1 hour to stain with the fluorescence. The nucleus was bound with DAPI (4',6-diamidino-2-phenylindole) attached with the fluorescence which was perchased from Sigma. After washing the cover glass, the specimen was prepared with the glass slide. The prepared specimen was analyzed with Confocal microscope.

### Example 6. Mitochondria Membrane Potential

3×10⁵ number of the young human fibroblast and the recovered aging cell were cultured in the culture dishes with 100 mm(φ) diameter, respectively. After the cells were treated with H₂O₂ for 6 hours, the cells were collected with Trypsin and then reacted with 80 nM of DiOC₆ (3,3-diethyloxacarbocyanine; Molecularprobes) at 37 °C for 30 minutes. After the cells were collected by centrifuging at 500 g for 5 minutes, the collected cells were added with the culture medium and then were analyzed with Flow cytometry. At least 10,000 cells were analyzed per one sample. The cells that were stained with DiOC₆ were identified with Channel FL1-H and the analyzed results were analyzed with Becton Dickinson FACS.

### Example 7. Immunoassay

Total cells were added into the buffer solution containing 10 mM of Tris-HCl pH 7.5, 1 mM of EDTA, 150 mM of NaCl, 1 % Triton X-100, 1 mM of protease inhibitor cocktail (Roche), 1 mM of phenylmethylsulfonyl fluoride (PMSF), 50 mM of sodium fluoride (NaF) and 0.2 mM of sodium vanadate (Na₃VO₄) to make a lysate. The lysate prepared from the above process was isolated with 10 % and 12 % SDS-PAGE, and then transferred into the nitrocellulose membrane to react with first antibody at 4 °C for 12 hours. The membrane attached with the first antibody was reacted with rat and rabbit secondary antibody that are bound with peroxidase for 1 hour to meet the conditions, and then was identified with Enhanced Chemiluminescence Detection Kit (Pierce Biotechnologies).

### Example 8. Thymidine Synthesizing

The human fibroblast was prepared by culturing to be 80 % of 24-well culture dish for performing three experiments. The cells were reacted with 1 mCi/mL of [methyl-3H] thymidine (2.0Ci/mmol; Amersham Biosciences, Buckinghamshire, UK) before collecting the cells. After removing the medium, the cells were twice washed with a cold PBS. The washed cells were reacted with a cold 10 % trichloroacetic acid (TCA) for 30 minutes, and then the DNA was precipitated by twice using the same method for each 5 minutes. After the cells were collected by reacting with the dissolving solution containing 0.5 N NaOH per one well for 5 minutes, the cells were added into 5 mL scintillation liquid to mix well with 5 N HCl. [methyl-3H] thymidine radio activity was measured with the machine.

### Example 9. Colonization

2.2 % agar solution was prepared by using PBS for making a soft agar and a hard agar. 0.75 ml of agar solution was mixed with 1.75 ml of DMEM containing 10 % serum extracted from a fetal bovine, 100 units/ml of penicillin, and 100 ug/ml of streptomycin. The mixed agar solution was added and hardened into the culture dish with 60-mm(φ) diameter to prepare a hard agar layer. 1 x 10⁴ cells were mixed with 0.5 ml agar solution and a soft agar prepared with 2 ml DMEM, and then poured and hardened on the hard agar layer. The state of cell culture was observed while the prepared agar was cultured in the incubator under the condition of 5% CO₂ and 37 °C for 12 ± 2 days.

### Example 10. Flow Cytometry

The young cell and aging cell were isolated from the dishes with Trypsin, and then reacted in a cold PBS containing 1 % BSA for 30 minutes. 1 x 10⁵ numbers of cells were mixed with PBS containing an integrin alpha V first antibody and 1 % BSA in the ratio of 1:50. After 1 hour, the human fibroblast was twice washed with PBS containing 1 % BSA. The washed cells were added into 100 ul of PBS containing 1 % BSA, with PE (phycoerythrin-group, B-phycoerythrin, Molecular Probes) secondary antibody. The results were analyzed with Becton Dickinson FACS.

### Example 11. Transduction of Ingegrin Alpha V siRNA

The ingegrin alpha V siRNA purchased from Dharmacon Research (Lafayette, Co. USA) was used for the experiment. siRNA SMARTpool duplex synthesized to order protected the expression of mRNA of the integrin alpha V. The integrin alpha V siRNA SMARTpool duplexes dissolved in 1 x siRNA buffer (diluted from 5 x siRNA buffer - Darmacon Product Cat. # B-002000-UB-100) was transducted into 2x10⁴ numbers of aging cells. Oligofectamine™ (Invitrogen) and siRNA were added into the medium without serum in order of transduction and then cultured in the incubator under the conditions of 5 % CO₂ and 37 °C for 4 hours. After 4 hours, the cells were added into the medium containing 10 % serum and then after 24, 48 and 72 hours, the cells were extracted.

### Example 13. DNA Fingerprint Analysis

The polymerase chain reaction was performed with the same amount of DNA. The polymerase chain reaction used AB Gene Amp PCR system 9700 Thermal Cycler (Applied Biosystems). The results obtained from the analysis were analyzed by using ABI Data Collection Software and GeneMapperTM 3.2.

### <Examples Results >

### 1. Stimulation of growth of aging cell by an extracellular matrix obtained from the young cell

The cell used for the experiment was the human fibroblast obtained from a foreskin. The inventors cultured the aging cell in the medium containing the extracellular matrix extracted from the young cell in order to identify whether the phenotype of cell is changed by an extracellular matrix or not. The young cell, of which the number of subculture was not higher than 26, was used, and the aging cell, of which the division did not occur anymore for 3 weeks after more than 60 of subcultures, was used. In addition, the inventors cultured each cell in the medium without serum for 3 days in order to obtain the extracellular matrix from the young cell and aging cell respectively. And, the cells was removed from the culture dishes by using EDTA (the cells were adhered to the culture dishes) and then the extracellular matrixes were obtained from the young cell and aging cell. The same numbers of the aging cells were added to the obtained extracellular matrixes and then the biological changes were observed.

The growth rate of the young cell in the culture dish with the extracellular matrix of aging cell seems to be reduced at first under the influence of the extracellular matrix as compared with the young cell in the general culture dish. However, it could be shown that it was back to the growth pattern similar to that of the young cell in the general culture dish after 4 days (Fig. 1 and Fig. 2). When the aging cell was cultured in the culture dish with the extracellular matrix of the young cell, it could be shown that the shape of aging cell was changed to that of young cell after 7 days. The growth rate of the cell that was changed to the young cell was rapidly increased. However, there was no change in the aging cell that was cultured in the culture dish with the extracellular matrix obtained from the aging cell (Fig. 3 and Fig. 4).

In order to identify whether the cell division of aging cell that was changed by the extracellular matrix obtained from the young cell occurred or did not, the aging cell was cultured in the culture dish with the young extracellular matrix and aging extracellular matrix for 5 days, the hourly variation was identified by using Time Lapse. From the results of observing the moving cells by using Time Lapse, it could be shown that the aging cell in the culture dish with the extracellular matrix obtained from the aging cell was not changed, while the aging cell in the culture dish with the extracelluar matrix obtained from the young cell was divided (Fig. 22 and Fig. 23). The aging cell with the flat-spreading shape in the normal stage was changed to the young cell with the spindling shape and was divided into the two daughter cells.

The above results prove that the original inherent growth ability of aging cell can be modulated as that of young cell by recovering the potential division ability through culturing in the culture medium with the extracellular matrix of the young cell, even though it is known that the aging cell cannot be divided and the growth ability of aging cell cannot be recovered.

### 2. Division Ability of the Recovered Aging Cell by the Extracellular Matrix Obtained from the Young Cell

The inventors performed Soft Agar Assay in order to identify whether the division ability obtained by culturing in the culture medium with the extracellular matrix is obtained from the change of the normal cell, or not. After the recovered aging cell was added into the soft agar, the cell was observed for 12 ± 2 days. Hela cell, which is a cancer cell as an experiment control, formed the colony after 2 weeks in the soft agar experiment. However, the recovered aging cell did not form the colony (Fig. 5). In addition, the recovered aging cell had the typical features of the aging cell as follows: the growth rate of the recovered aging cell was again decreased; its shape was changed to the flat shape; and it was reacted with a beta galactosidase, after 20 to 25 numbers of subcultures (Fig. 6).

The above results prove that the recovery of the growth ability of the recovered aging cell is not caused by the cancer change of the aging cell.

### 3. The Cellular Shape of the Aging Cell Recovered by the Extracellular Matrix Obtained from the Young Cell.

The aging cell recovered by the extracellular matrix obtained from the young cell has the features of the normal young cell as it is. It could be known that the features of the recovered aging cell were similar to those of the young cell from the results of identifying through a beta galactosidase staining (First Panel), CM-H₂DCFDA staining (Second Panel) and F-actin staining (Third Panel), which are well known as the marker of aging cell, in order to identify whether there is an aging cell or not (Fig. 7).

According to the previous study, it could be known that when the aging of the cell proceeds, the mitochondria potential is decreased (23). Thus, the mitochondria membrane potential was measured as follows: the young cell, the aging cell and the recovered aging cell were treated with 1.5 mM of H2O2; after 6 hours, the above cells were reacted with 80 nM of DiOC6 for 30 minutes; and then the survival rate of the cells were measured at 488 nm of absorbance, like the method similar to the method in the previous documents. From the above results, it could be shown that the value of the mitochondria membrane potential in the recovered aging cell after adding H2O2 was similar to the value of the membrane potential of the young cell. However, there were no reactions in the aging cell (Fig. 8).

It is known that when the cell is aged, p21Waf1 and p16INK4a, which are a defense molecular of the cell cycle and control the cell division, are increased (24, 25). The inventors found out that a caveolin-1 that is increased when the cell is aged in the previous documents defends the growth activity that is depended on EGF (26). Thus, the inventors identified the effects of the factors, which affect in the cell cycle and growth, on the recovered aging cell. From the results, it could be shown that the amounts of protein expressions of p21Waf1, p16INK4a and a caveolin were decreased (Fig. 9) and Erk phosphorylation was occurred when giving EGF stimulation like the young cell (Fig. 10). From the previous results, it could be identified that the aging cell was recovered to the young cell by the extracellular matrix obtained from the young cell.

### 4. Integrin Alpha V odulating the Activity of Cell Growth

The inventors compared the expression degrees of integrin in the young cell and aging cell through Flow cytometry. From the results as disclosed in Fig. 4a and Fig. 4b, it could be known that the degree of binding and amount of the protein expression of integrin alpha V that is directly bound to the cell when aging the cell were decreased. It is known that the integrin alpha5beta1 that is bound to the fibronectin that is an extracellular matrix is an important factor in aging(28). Thus, the inventors identified through Flow cytometry in order to identify whether the cell used for the present invention has a same result or not, and then it could be found that there was no difference between the degree of expression of the integrin alpha5beta1 in the aging cell and that in the young cell in the case of the human fibroblast (Fig. 12). When the amount of expression of integrin alpha5beta1 was artificially decreased by using the antibody in the young cell and aging cell, the cells did not adhere to the culture dish. From the above results, it could be known that the integrin alpha5beta1 that is bound to the fibronectin plays an important role in binding in the human fibroblast regardless of aging.

In order to identify an exact role of integrin alpha V in the process of cell division, the inventors measured the number of cells and the binding degree of 3H-thymidine after adding the defense antibody to the young cell (Fig. 14). It is known that the integrin alpha(V)beta3 and integrin alpha(V)beta5 affect the cell division, a cancer formation and a function of blood vessel (29). The inventors identified the ability of cell division of the young cell by using the defense antibodies of the integrin alpha(V)beta3 and the integrin alpha(V)beta5 in order to identify the ability of the cell division, and then found that the ability of cell division was decreased. In addition, when using both defense antibodies at the same time, the ability of cell division was surely decreased as compared with the case of using one defense antibody (Fig. 13). From the above experiments, the result could be obtained that both of the degree of synthesizing DNA and the ability of the cell division of the human fibroblast were equally decreased by the defense antibody of the integrin alpha V.

### 5. Change of Integrin Alpha V in the Process of Recovering Aging Cell

In order to identify an exact role of integrin alpha V, the amount of expression of integrin alpha V was identified through Western Blot in the process of recovering aging cell. After adding the aging cell to the extracellular matrix obtained from the young cell, the cells were extracted per day and then the amount of expression of integrin alpha V was measured. Interestingly, for the cells obtained from the aging cell that was added to the young extracellular matrix at 1, 2, 3, 10 and 14 days, the amounts of expressions of integrin alpha V were the same as the amount of expression of the young cell. From the above result, it could be known that the amount of expression of integrin alpha V was the same as that of young cell, even though it could not change to the young cell morphologically in the case of the aging cell that was added to the young extracellular matrix (Fig. 15).

In order to clearly identify the role of integrin alpha V when recovering the aging cell by the extracellular matrix obtained from the young cell, the amount of integrin alpha V was artificially decreased by using siRNA and antibody and then the growth rate and degree of synthesizing DNA of cell were identified. When adding the defense antibody, the aging cell was not recovered by the young extracellular matrix (Fig. 16 and Fig. 17). In order to more clarify one more time, the experiment was performed by using siRNA in order to modulate the gene expression of the integrin alpha V. From the result, it could be known that the aging cell shown the recovery feature in the extracellular matrix obtained from the young cell, but when the amount of expression was artificially decreased by using siRNA and defense antibody of integrin alpha V, the aging cell was not recovered to the young cell (Fig. 18, Fig. 19 and Fig. 20).

### <Discussion>

The human fibroblast becomes the aging state that could not be changed after the certain numbers of cell divisions. It is known that the changed aging cell has a dominant property as compared with the young cell and cancer cell. However, the inventors obtained the conclusion that the aging cells can be recovered to the young cell by the extracellular matrix obtained from the young cell according to the present invention. Thus, the inventors found the problem in the concept of aging that is well known as the unchangeable aging cell in the conventional concept.

It is well known that the extracellular matrix controls the binding and cell division of cell, and the survival of the cancer cell and stem cell. It is thought that the components of the extracellular matrix are a key factor for the process of aging, even though the mechanism of aging is not known. The inventors obtained the conclusion that the extracellular matrix that is not known can change the aging feature of cell according to the present invention. As shown in Fig. 1, the decreased function of the aging cell was recovered by the affect of the extracellular matrix obtained from the young cell and also the morphology of the aging cell could be changed to that of the young cell. However, the aging cell in the extracellular matrix obtained from the aging cell was not changed. From the above result, it could be known that the extracellular matrix obtained from the young cell plays an important role in changing the function of the cell. At first, the adding of the young cell to the extracellular matrix obtained from the aging cell affected the growth of the cell. However, the growth rate of the above cell became the rate similar to the growth rate of the normal young cell that was used for the control within 4 days. From the above result, it can be predicted that the properties of their components and the extracellular matrixs obtained from the young cell and aging cell are different. The human fibroblast obtained from a child was added to the extracellular matrix of the young cell from the newborn baby in order to prove that the aging cell recovered by the extracellular matrix obtained from the young cell is not the cell used as the extracellular matrix. From the above result, it could be shown that the extracellular matrix of the young cell obtained from the newborn baby and the extracellular matrix of the human fibroblast obtained from a child show a same effect (Fig. 24 and Fig. 25). DNA fingerprinting was used for identifying that the aging cell recovered by the extracellular matrix obtained from the newborn baby has a same gene as that of the child fibroblast. As shown in Fig. 24 and Fig. 25, the young cell obtained from a child, the aging cell that is obtained from the repetitive subculture of the above young cell, and the aging cell recovered by the extracellular matrix of the young cell had the same allelic gene. However, the cell obtained from the newborn baby that is used as the extracellular matrix had the allelic gene different from the above gene. It could be identified that the aging cell can be recovered regardless of the species from the previous results that all of the aging cells can be recovered by the extracellular matrix obtained from the newborn baby and the young cell. In addition, the recovery of the aging cell was exactly identified through a microscope per hour (Figs. 21 to 23). The gene pattern of the aging cell is almost similar to that of the young cell through identifying by using Gene Analysis (Figs. 26 to 29). From the previous results, it could be identified that the aging cell can be recovered as the young cell according to the control of the culture conditions.

From the previous thesis, the inventors identified that the functional and morphological recovery can be partly performed, but not completely by controlling a caveolin-1 (12). The caveolin-1, of which the amount of the expression is increased in the aging cell, controls the reaction of the growth factor (26). When the amount of the expression of the caveolin-1 is artificially decreased by using siRNA, the reaction of the growth factor and also the degree of synthesizing DNA in the aging cell were increased and then its shape was also changed. In addition, the phenotype of aging depending upon p52/p21 is recovered as the young cell when p53 was not activated (33).

Thus, it required that whether a cancer is activated or not when controlling the caveolin-1 and p53 that are well known as the tumor suppressor in the aging cell. According to the present invention, the amount of the expression of the molecules, which defense the cell cycle and p53 in the aging cell recovered by the extracellular matrix extracted from the young cell, was decreased. In addition, the ability of forming the colony was identified by using Hela cell as a control in order to identify whether the recovered aging cell has a growth ability that can not be controlled by themselves, or not. From the result, the recovered aging cell did not form the colony, but Hela cell that was used as a control formed the colony (Figs. 15 to 20). The recovered aging cell did not react to a beta galatosidase, and the accumulation of its activated oxygen was reduced, and the reduced F-actin staining was increased (Figs. 7 to 10). In addition, it could be found that the gene patterns of the recovered aging cell and the young cell are similar to each other through Gene Analysis. The above results mean that the recovered aging cell, which has an increased cell division by the extracellular matrix obtained from the young cell, was recovered as the normal young cell.

Curiously, the aging cell that was similarly recovered as the type of young cell was back to the stage of aging after several time of sub-culturing. The inventors found that the ability of the cell division of the recovered aging cell was changed after 20 times of sub-culturing, and then back to the aging showing the reaction to the beta galactosidase (Fig. 6). It is a normal phenomenon that the aging cell recovered by the young extracellular matrix is back to the aging. The mechanism is not identified exactly and is required in the further for the study for recovering of aging cell. In order to identify the key factors as disclosed in the previous researches, the inventors focused on the integrin that binds to the extracellular matrix. Thus, the inventors compared the amounts of expressions of the integrins between the young human fibroblast and the aging cell.

The integrin is a receptor that is present in the cell membrane binding to the extracellular matrix that is composed of alpha and beta chains, composes the cell frame, and controls the survival, growth, division and movement as the signaling in the cell. According to the present invention, the inventors found that the integrin alpha V was reduced when aging, and was a important factor for controlling the growth of cell in the aging cell recovered by the extracellular matrix extracted from the young cell, and the young cell (Figs. 11 to 14 and 15 to 20). Based on the previous results, it could be predicted that the integrin alpha V binding to the extracellular matrix of the young cell stimulates the recovery of the physiological cell division of the aging cell.

It is known that the integrin alpha V plays an important role on the differential division as the constituent of integrin. The rat without the integrin alpha V dies soon after being born, and it is known that when integrin alpha V is knockout, the rat has problems such as the formation of abnormal embryo including a brain, and the formation of abnormal intestine blood vessel (34). According to the present invention, it is thought that the integrin alpha V plays an important role on the recovery of the aging cell that is caused by the extracellular matrix extracted from the young cell. The result may help the study for identifying the effect and the new function of the extracellular matrix that is involved in signaling of the cell growth.

### <Reference>

1. Hayflick L, Moorhead PS, The serial cultivation of human diploid cell strains, Exp Cell Res. 25, (1961) 585-621.
2. Smith JR, Lincolon DW, Aging of cells in culture, Int Rev Cytol. 89, (1984)151-77.
3. Goldstein S, Replicative senescence: the human fibroblast comes of age, Science. Rev. 249, (1990): 1129-33.
4. Warner HR, Campisi J, Cristofalo VJ, Miller RA, Papaconstainou J, Pereira-Smith O, Smith JR, Wang E, Control of cell proliferation in senescent cells, J. Gerontol. 47, (1992) B185-9.
5. Cristofalo VJ, Pignolo RJ, Replicative senescence of human fibroblast-like cells in culture, Physiol Rev. 1993 Jul;73(3):617-38. Review.
6. Chang BD, Xuan Y, Broude EV, Zhu H, Schott B, Fang J, Roninson IB, Role of p53 and p21waf1/cip1 in senescence-like terminal proliferation arrest induced in human tumor cells by chemotherapeutic drugs, oncogene. 34, (1999) 4808-4818.
7. Kagawa S, Fujiwara T, Kadowaki Y, Fukazawa T, Sok-Joo R, Roth JA, Tanaka N, overexpression of the p21 sdi1 gene induces senescence-like state in human cancer cells: implication for senescence-directed molecular therapy for cancer, Cell Death Differ. 6, (1999) 765-772.
8. Ohkusu-Tsukada, K., Tsukada, T., and Isobe, K, Accelerated development and aging of the immune system in p53-deficient mice, J. Immunol. 15, (1999) 1966-1972
9. Ryu SJ, Cho KA, Oh YS, Park SC, Role of Src-specific phosphorylation site on focal adhesion kinase for senescence-associated apoptosis resistance. Apoptosis. 2006 Mar;11 (3):303-13.
10. Cho KA, Ryu SJ, Oh YS, Park JH, Lee JW, Kim HP, Kim KT, Jang IS, Park SC, Morphological adjustment of senescent cells by modulating caveolin-1 status. J Biol Chem. 2004 Oct 1;279(40):42270-8.
11. Dimri GP, Lee X, Basile G, Acosta M, Scott G, Roskelley C, Medrano EE, Linskens M, Rubelj I, Pereira-Smith O, A biomarker that identifies senescent human cells in culture and in aging skin in vivo. 1995 Sep 26;92(20):9363-7
12. Cho KA, Ryu SJ, Park JS, Jang IS, Ahn JS, Kim KT, Park SC. Senescent phenotype can be reversed by reduction of caveolin status. 2003 Jul 25;278(30):27789-95. Epub 2003 May 1
13. Robert L. Biology of aging Rev Prat. 1993 Oct 15;43(16):2104-11. Review.
14. Kern P, Moczar M, Robert L. Biosynthesis of skin collagens in normal and diabetic mice. Biochem J. 1979 Aug 15;182(2):337-45.
15. Rasoamanantena P, Thweatt R, Labat-Robert J, Goldstein S. Altered regulation of fibronectin gene expression in Werner syndrome fibroblasts. Exp Cell Res. 1994 Jul;213(1):121-7.
16. Krtolica A, Parrinello S, Lockett S, Desprez PY, Campisi J. Senescent fibroblasts promote epithelial cell growth and tumorigenesis: a link between cancer and aging. Proc Natl Acad Sci USA. 2001 Oct 9;98(21):12072-7. Epub 2001 Oct 2.
17. Krtolica A, Parrinello S, Lockett S, Desprez PY, Campisi J. Senescent fibroblasts promote epithelial cell growth and tumorigenesis: a link between cancer and aging. Proc Natl Acad Sci USA. 2001 Oct 9;98(21):12072-7. Epub 2001 Oct 2.
18. Hynes RO, Integrins: bidirectional, allosteric signaling machines. 2002 Sep 20;110(6):673-87.
19. Hood JD, Cheresh DA, Role of integrins in cell invasion and migration. Nat Rev Cancer. 2002 Feb;2(2):91-100. Review
20. Leroy-Dudal J, Demeilliers C, Gallet O, Pauthe E, Dutoit S, Agniel R, Gauduchon P, Carreiras F, Transmigration of human ovarian adenocarcinoma cells through endothelial extracellular matrix involves alphav integrins and the participation of MMP2. 2005 Apr 20;114(4):531-43.
21. Brooks PC, Str 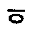 mblad S, Klemke R, Visscher D, Sarkar FH, Cheresh DA. Antiintegrin alpha v beta 3 blocks human breast cancer growth and angiogenesis in human skin. J Clin Invest. 1995 Oct;96(4):1815-22.
22. Clezardin P, Recent insights into the role of integrins in cancer metastasis. Cell Mol Life Sci. 1998 Jun;54(6):541-8. Review.
23. Ryu SJ, Oh YS, Park SC. Failure of stress-induced downregulation of Bcl-2 contributes to apoptosis resistance in senescent human diploid fibroblasts. Cell Death Differ. 2007 May;14(5):1020-8. Epub 2007 Feb 9.
24. McConnell BB, Starborg M, Brookes S, Peters G. Inhibitors of cyclin-dependent kinases induce features of replicative senescence in early passage human diploid fibroblasts. Curr Biol. 1998 Mar 12;8(6):351-4.
25. Stein GH, Drullinger LF, Soulard A, Duli V. Differential roles for cyclin-dependent kinase inhibitors p21 and p16 in the mechanisms of senescence and differentiation in human fibroblasts. Mol Cell Biol. 1999 Mar;19(3):2109-17.
26. Park WY, Park JS, Cho KA, Kim DI, Ko YG, Seo JS, Park SC. Up-regulation of caveolin attenuates epidermal growth factor signaling in senescent cells. J Biol Chem. 2000 Jul 7;275(27):20847-52.
27. Clark EA, Brugge JS. Integrins and signal transduction pathways: the road taken. Science. 1995 Apr 14;268(5208):233-9. Review.
28. Labat-Robert J. Cell-matrix interactions in aging: role of receptors and matricryptins. Ageing Res Rev. 2004 Apr;3(2):233-47. Review.
29. Erdreich-Epstein A, Shimada H, Groshen S, Liu M, Metelitsa LS, Kim KS, Stins MF, Seeger RC, Durden DL. Integrins alpha(v)beta3 and alpha(v)beta5 are expressed by endothelium of high-risk neuroblastoma and their inhibition is associated with increased endogenous ceramide. Cancer Res. 2000 Feb 1;60(3):712-21.
30. Bunn CL, Tarrant GM, Limited lifespan in somatic cell hybrids and cybrids. 1980 Jun;127(2):385-96.
31. Muggleton-Harris AL, DeSimone DW, Replicative potentials of various fusion products between WI-38 and SV40 transformed WI-38 cells and their components. 1980 Nov;6(6):689-98.
32. Pereira-Smith OM, Smith JR, Expression of SV40 T antigen in finite life-span hybrids of normal and SV40-transformed fibroblasts. 1981 Jul;7(4):411-21.
33. Christian M. Beaus jour, Ana Krtolica, Francesco Galimi, Masashi Narita, Scott W. Lowe, Paul Yaswen, Judith Campisi, Reversal of human cellular senescence: roles of the p53 and p16 pathways. 2003 Aug 15;22(16):4212-22.
34. Bader BL, Rayburn H, Crowley D, Hynes RO. Extensive vasculogenesis, angiogenesis, and organogenesis precede lethality in mice lacking all alpha v integrins. Cell. 1998 Nov 13;95(4):507-19.

## Claims

1. A composition containing an extracellular matrix component for controlling aging.

2. The composition for controlling aging of claim 1, wherein the extracellular matrix is obtained from the cell culture.

3. The composition for controlling aging of claim 2, wherein the cell is originated from a mammal.

4. The composition for controlling aging of claim 3, wherein the cell is a human fibroblast.

5. The composition for controlling aging of any one of claims 1 to 4, wherein the extracellular matrix component is originated from the young cell.

6. The composition for controlling aging of claim 1, further comprising siRNA to an integrin alpha V.

7. The composition for controlling aging of claim 1, further comprising an antibody to an integrin alpha V.

8. A method for controlling aging, comprising treating an object with a composition containing an extracellular matrix component.

9. The method for controlling aging of claim 8, comprising obtaining an extracellular matrix by culturing a young cell; and culturing by inoculating the aging cell that is not divided any more in the culture medium containing the above extracellular matrix obtained.

10. The method for controlling aging of claim 9, wherein the young cell is a human fibroblast.

11. A composition for controlling aging, comprising at least two of a ligand, siRNA, and an antibody to the integrin alpha V.

12. The composition for controlling aging of claim 11, wherein the ligand is selected from the group consisting of a vitronectin, a fibronectin and their analogues.

13. A method for controlling aging, comprising artificially reducing the amount of expression of the integrin alpha V by treating an object with siRNA to the integrin alpha V.

14. A method for controlling aging, comprising artificially reducing the activity of the integrin alpha V by treating an object with the antibody to the integrin alpha V.
